# EUROPEAN PATENT SPECIFICATION

(11) **EP 3 402 442 B1**
(45) Date of publication and mention of the grant of the patent: **11.03.2026**
(21) Application number: 17738908.7
(22) Date of filing: 12.01.2017
(51) Int. Cl.: A61F 2/34, A61F 2/30, A61F 2/46

(54) **ORTHOPAEDIC IMPLANTS WITH TEXTURED POROUS SURFACES**
ORTHOPÄDISCHE IMPLANTATE MIT TEXTURIERTEN PORÖSEN OBERFLÄCHEN
IMPLANTS ORTHOPÉDIQUES À SURFACES POREUSES TEXTURÉES

(30) Priority: 12.01.2016 US 201662277755 P
(43) Date of publication of application: 21.11.2018
(73) Proprietor: SMed - TA/TD LLC, Columbia City, IN 46725 (US)
(72) Inventor: JURICK, Joseph, W., Fort Wayne IN 46818 (US); NEBOSKY, Paul, S., Fort Wayne IN 46804 (US); STALCUP, Gregory, C., Columbia City IN 46814 (US)
(74) Representative: Sawodny, Michael-Wolfgang
(86) International application number: PCT/US2017/013147
(87) International publication number: WO 2017/123724

(56) References cited:
- EP-A1- 2 869 787
- US-A- 5 348 788
- US-A- 5 496 372
- US-A1- 2006 235 542
- US-A1- 2011 251 698
- US-A1- 2013 331 893
- US-A1- 2014 138 010
- US-A1- 2014 277 461
- US-A1- 2015 257 871

## Description

### BACKGROUND OF THE INVENTION

### 1. Field of the Invention

The present invention relates to orthopaedic implants, and, more particularly, to orthopaedic implants incorporating porous materials. The closest prior art is document US 5348788 A, which defines the preamble of claim 1.

### 2. Description of the Related Art

Orthopaedic implants are medical devices used for replacing or providing for stabilization and fixation of a bone or for replacement of articulating surfaces of a joint. The need for surgery requiring the implantation of such a medical device is usually the result of osteoarthritis, also known as degenerative joint disease, or injury. In the past, such orthopaedic implants have been formed of a solid, biocompatible material, which have been utilized with the goal of giving the patient an improved quality of life with reduced pain and inflammation, as well as increased stability, mobility and directed flexibility.

After implanting an orthopaedic implant into a patient, one of the most common causes of implant failure occurs due to insufficient fixation of the implant. Especially in implants at joints, where there are generally multiple moving anatomy features adjacent the implant, the implant being insufficiently fixated can cause movement of the implant from its correct positioning and/or orientation. When the positioning and/or orientation of the implant is incorrect, the load-bearing characteristics of the implant can be altered to such a degree that the implant fails due to material fracture and/or the implant failing to bear sufficient load from adjacent tissue. Regardless of the implant failure mode, a revision or replacement surgery is typically necessary to correct the issues caused by the implant failing to sufficiently fixate.

A known way for increasing implant fixation is to provide the implant with one or more porous materials having many pores which encourage surrounding ingrowth of tissue into the pores. The tissue growing into the pores helps adhere the implant to the implantation site. reducing the risk of implant failure. To further promote tissue ingrowth into the pores, the pores may be pre-filled with one or more biological substances such as growth factors and/or stem cells prior to implantation. While pre-filling the pores with such biological substances can increase the tissue ingrowth volume and rate into the pores, the rate of tissue ingrowth into the pores is still quite slow and is often insufficient to allow the implant to bear load from surrounding anatomical structures for several weeks, if not months, following implantation. During this time, the patient should not load the implantation area due to the significant risk of implant failure due to insufficient fixation.

What is needed in the art is an orthopaedic implant can address some of the previously described disadvantages of known implants.

### SUMMARY OF THE INVENTION

The present invention is defined in claim 1 and provides an orthopaedic implant with a textured porous material having a plurality of islands which are configured to shear biological tissue during implantation.

The invention may be used in a method of implanting an orthopaedic implant including an implant body with an outer surface and a textured porous material attached to the outer surface and having a plurality of pores and a plurality of islands configured to shear biological tissue during implantation, which includes: preparing an anatomical site to accept the orthopaedic implant; and pressing the orthopaedic implant into the prepared anatomical site.

An advantage of the present invention is the islands can shear biological material, such as tissue, during implantation to provoke the natural healing response and increase the ingrowth rate of tissue into the pores.

Another advantage is the orthopaedic implant can be implanted using known surgical techniques, increasing the chance of physician adoption.

Yet another advantage is the porous textured material can be filled by autologous cells, tissues, and/or substances during implantation to increase the ingrowth rate of tissue into the pores with minimal risk of autoimmune reactions.

### BRIEF DESCRIPTION OF THE DRAWINGS

The above-mentioned and other features and advantages of this invention, and the manner of attaining them, will become more apparent and the invention will be better understood by reference to the following description of embodiments of the invention taken in conjunction with the accompanying drawings, wherein:
Fig. 1 is a perspective view of an embodiment of an orthopaedic implant formed according to the present invention;
Fig. 2 is a perspective view of the orthopaedic implant shown in Fig. 1 with an additional set of helical grooves;
Fig. 3 is a close-up view of a textured porous material formed according to the present invention;
Fig. 4 is a microscopic view of another embodiment of a textured porous material formed according to the present invention;
Fig. 5 is an additional microscopic view of the textured porous material shown in Fig. 4;
Fig. 6 is a perspective view of the orthopaedic implant shown in Fig. 1 being implanted in a patient; and
Fig. 7 is a cross-sectional view of the orthopaedic implant shown in Fig. 1 after being implanted in a patient such that some of pores of the textured porous material are filled with biological material.

Corresponding reference characters indicate corresponding parts throughout the several views. The exemplifications set out herein illustrate embodiments of the invention and such exemplifications are not to be construed as limiting the scope of the invention, which is defined by the claims.

### DETAILED DESCRIPTION OF THE INVENTION

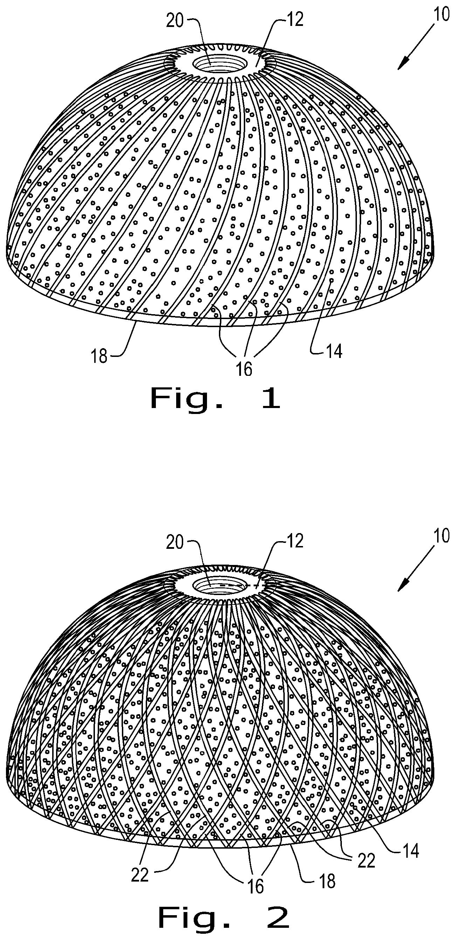

Referring now to the drawings, and more particularly to Figs. 1-2, embodiments of an orthopaedic implant 10 according to the present invention are shown and generally include an implant body 12, shown as a semi-spherical acetabular cup, with a textured porous material 14 covering the outer surface of the implant body 12. While the implant body 12 is shown as an acetabular cup, it should be appreciated that any shape of implant body can be used according to the present invention. The implant body 12 can be formed of any biocompatible material that is suitable for short or long term implantation in an animal or human organism. Suitable biomaterials can include, but are not limited to: metals such as titanium, tantalum, stainless steel, and cobalt chrome; polymers such as polyether ether ketone (PEEK) or polyaryl ether ketones (PAEK) generally, various molecular weight polyethylene (PE), polylactic acid (PLA), and polyglycolic acid (PGA); and other materials such as bioceramics, bioglasses, hydroxyapatite, and composite materials. The implant body 12 can be substantially non-porous, i.e., solid, or have pores formed in the body. As shown, the textured porous material 14 is posited on the non-porous material of the acetabular cup 12 with grooves 16 formed in the textured porous material 14 to prevent rotation of the acetabular cup 12 following implantation. As shown in Fig. 1, the grooves 16 are helical and extend from a bottom 18 of the cup 12 to an apex 20 of the cup 12. As shown in Fig. 2, the grooves 16 are also helical extending from the bottom 18 to the apex 20, and intersecting helical grooves 22 are also formed in the porous material 14 that are directed in the opposite direction to grooves 16 and cross the grooves 16 to form diamond shapes in the porous material 14. It should be appreciated that the grooves 16, 22 are optional and may not be desired in some embodiments of implants.

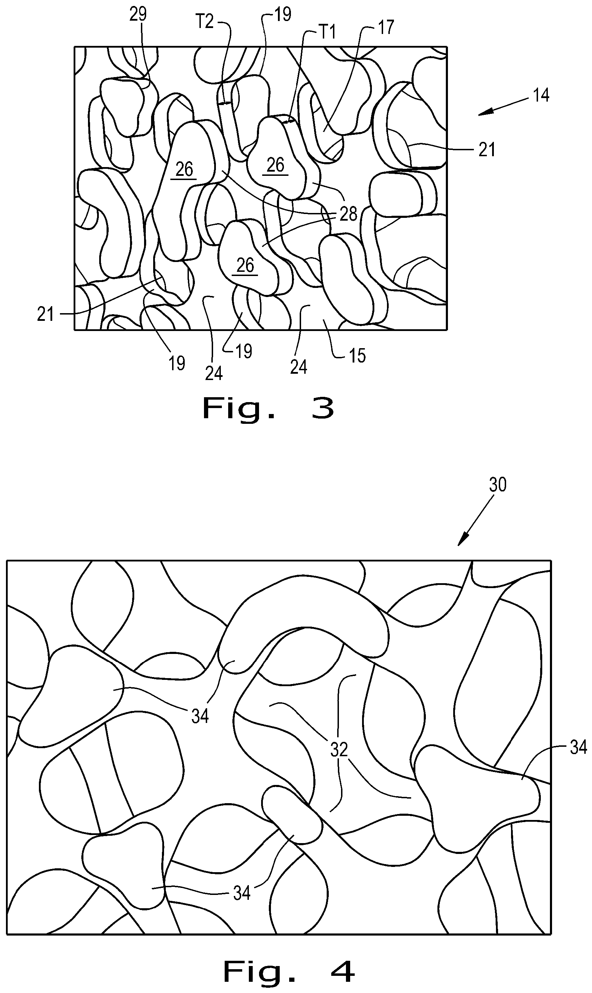

Referring now to Fig. 3, a close-up view of a portion of the textured porous material 14 is shown. The textured porous material 14 includes multiple porous material layers, such as a first porous material layer 15 and a second porous material layer 17, that are bonded together, with each bonded layer having a pore pattern formed therein. The first porous material layer 15 includes a first plurality of pores 19 and define an outer porous layer, i.e., the outermost porous material layer, and the second porous material layer 17 can include a second plurality of pores 21 and be between the outer porous layer 15 and the outer surface of the implant body 12. As can be seen in Fig. 3, the pore patterns may not be identical or completely overlapped, in effect causing the overlap of pores 19 in one layer 15 with pores 21 of one or more adjacent layers 17 to define a pore that extends through multiple layers 15, 17 of the porous material 14 and has a shape defined by the overlap of the two overlapping pores 19, 21. The pores 19, 21 can be formed in the porous material 14, or each individual layer 15, 17 of the porous material 14, by any suitable method, such as laser cutting, chemical etching, punching, etc. Each pore formed in a layer is defined as being surrounded by interconnecting struts 24, with the struts 24 defining the material portion of the porous material 14. While only the struts 24 of the first porous material layer 15 are numbered, it should be appreciated that the second porous material layer 17 and other porous material layers, if included, also include a plurality of struts with the pores 21 defined by the struts. The struts 24 can comprise the same or different biocompatible material as the implant body 12, with the previously described biomaterials also being suitable materials for the struts 24.

During formation of the porous material 14, a texture is imparted to the outermost surface 15 of the porous material 14 that shears bone material or other biological tissue(s) at the implantation site and direct the sheared tissue(s) into one or more of the pores 19, 21 formed in the porous material 14 during the implantation procedure, packing tissue(s) and other biological materials, such as blood and stem cells, into some or all of the pores 19, 21. The sheared tissue can also be referred to as "uncultured biological material," since the sheared tissue is formed of cells and other biological materials which have not been cultured in any environment other than in vivo. By packing one or more pores 19, 21 of the porous material 14 with uncultured biological material, such as recently sheared bone material, blood, stem cells, etc., the orthopaedic implant 10 can fixate to surrounding bone tissue in a relatively fast timeframe compared to non-textured implants, even those which have cultured biological material packed in the pores prior to implantation. As used herein, the term "recently sheared" biological material is biological material that has been separated from its in vivo source within a timeframe of roughly 1-5 seconds. The exact cause of the improved fixation is currently being investigated, but it is hypothesized that packing the pores 19, 21 with recently sheared tissue and other biological material that is very recently collected synergistically combines with provoking the body's natural repair response at the surface of the sheared anatomical feature, such as bone, to cause rapid ingrowth of body tissue into the pores 19, 21 of the porous material 14 which fixates the implant 10. Packing the pores 19, 21 of the porous material 14 with recently sheared biological material by shearing a bone that the implant 10 rubs against, therefore, is believed to simultaneously produce an implant 10 which is well-prepared for promoting bone ingrowth into the pores 19, 21 for fixation by virtue of the pores 19, 21 being filled with tissue ingrowth promoting substances and an environment at the implantation site which is conducive for fixating the implant 10 to bone.

To impart a texture on the outermost surface of the porous material 14 according to the present invention, islands 26 of material are formed on or attached to the struts 24 of the outermost material layer 15 to form the texture on the outermost surface of the porous material 14. Unlike the struts 24, which are connected to one another and define the pores 18, 21 therebetween, the islands 26 are disconnected from each other and define raised shearing surfaces, similar to the surface of a grater. To better shear biological tissue during implantation, the islands 26 are formed of a shearing material with a hardness greater than cortical bone, i.e., the shearing material will scratch cortical bone tissue when scraped across cortical bone tissue. The islands 26 can have many different shapes across the surface of the porous material 14, as shown, and the distribution of the shapes can be random or follow a pre-determined pattern if desired. As shown in Fig. 3, each island 26 can be formed as a thickened portion of an individual strut 24 that does not overlap with the pores 19 formed in the outermost layer 15. The islands 26 can each have peripheral surfaces 28 defining one or more curvatures so the islands 26 have curved peripheral surfaces that not only apply shearing force to the bone as the implant 10 is pressed against the bone, but also direct the sheared material toward the pores of the porous material 14. If desired, one or more of the islands 26 can also have peripheral surfaces that define linear angles, i.e., are flat. Beveled edges, such as edge 29, can also be formed in the islands 26 in order to more effectively shear bone material that the island 26 rubs against during implantation. It should be appreciated that the islands 26 do not need to cover an entirety of the outermost surface of the porous material 14, but may only cover a portion of the porous material 14 where shearing of bone material to pack the adjacent pores is desired. Similarly, the coverage of the struts 24 of the porous material 14 by the islands 26, as a percentage, can be varied in different regions of the porous material 14. It may also be desired to form individual islands 26 with multiple thicknesses to produce an uneven face on the island and/or form the islands 26 with varying thicknesses, relative to each other, to form an uneven texture on the porous material 14.

To form the islands 26, the islands 26 can be formed in a separate material layer attached to what will be the outermost layer 15 of the porous material 14 having struts 24 or the islands 26 can be formed as an integral part of the outermost layer having struts 24. For example, the islands 26 can be formed from a layer of island material that is bonded to what will eventually be the outermost layer 15 of the porous material 14 having struts 24. The island material layer can be bonded to the outermost layer 15 of the porous material 14 with an intermediate protective layer between the island material layer and the outermost layer 15 of the porous material 14. The desired pattern of islands 26 can then be photo or chemical etched into the island material layer, with the intermediate protective layer protecting the material of the outermost layer 15 of the porous material 14 from being etched. After the islands 26 are formed, the protective layer can be washed away and the pore pattern can then be formed in the outermost layer 15 of the porous material 14 to produce the struts 24 and pores 19, 21. The islands 26 can also be formed, for example, by additive manufacturing (also known as "3D printing") the outermost layer 15 of the porous material 14. It should be appreciated that the described manufacturing techniques are exemplary only, and the texture, whether formed of islands 26 or otherwise, can be imparted to the outermost surface of the porous material 14 in any suitable fashion. Further, the islands 26 can each have an island thickness T1 which is greater than a first layer thickness T2, defining an average thickness of the struts 24 defining the material of the layer 15, of the outermost layer 15 so the islands 26 extend away from the outer surface of the implant body 12 to shear biological tissue as the implant 10 is implanted. The first layer thickness T2, for example, may be no more than 50 to 100 microns while the island thickness T1 of the islands 26 can be 150 microns or greater. Further, the islands 26 may be formed to have no spatial dimension, i.e., width, thickness, or length, which is greater than 600 microns.

Referring now to Figs. 4-5, an alternative embodiment of a textured porous material 30 formed according to the present invention is shown. As can be seen, the porous material 30 is formed of bonded porous material layers 31, 33 having struts 32 and pores 35, 37 formed therein. Islands 34 are also connected to the struts 32 to form the texture, but unlike the porous material 14 shown in Fig. 3, the islands 34 of the textured porous material 30 can overlap with pores 35, 37 formed in the material layers 31, 33 of the porous material 30. In such an embodiment, the islands 34 are not merely increased thicknesses of the struts 32, but are attached to the struts 32 in order to shear biological material, such as bone material, and direct the sheared bone material into the pores 35, 37 of the porous material 30. Overlapping material of the islands 34 with the pores 35, 37 can be useful, for example, to increase the total surface area of the islands 34 in aggregate and produce a less coarse texture on the porous material 30.

From the foregoing description, it should be appreciated that the texture can be formed on the outermost surface of the porous material of an implant in a variety of ways. While the texture is described as multiple islands that are not connected to one another, the formed islands can be connected to one or more adjacent islands to form the texture. Further, the texture formed on the outermost surface of the porous material does not need to be the same across the outermost surface, but distinct regions with differing textures can be formed on the outermost surface. For example, the porous material 14 shown in Figs. 1-2 may have a region with a coarser texture near the apex 20 of the cup 12 and another region with a finer texture near the bottom 18 of the cup 12. Variations in texture across the outermost surface of the porous material 14 can allow for different patterns of shearing in the biological material, such as bone material, as the implant 10 is being implanted, which can help control the degree of the body's natural repair response in various regions of the implantation site.

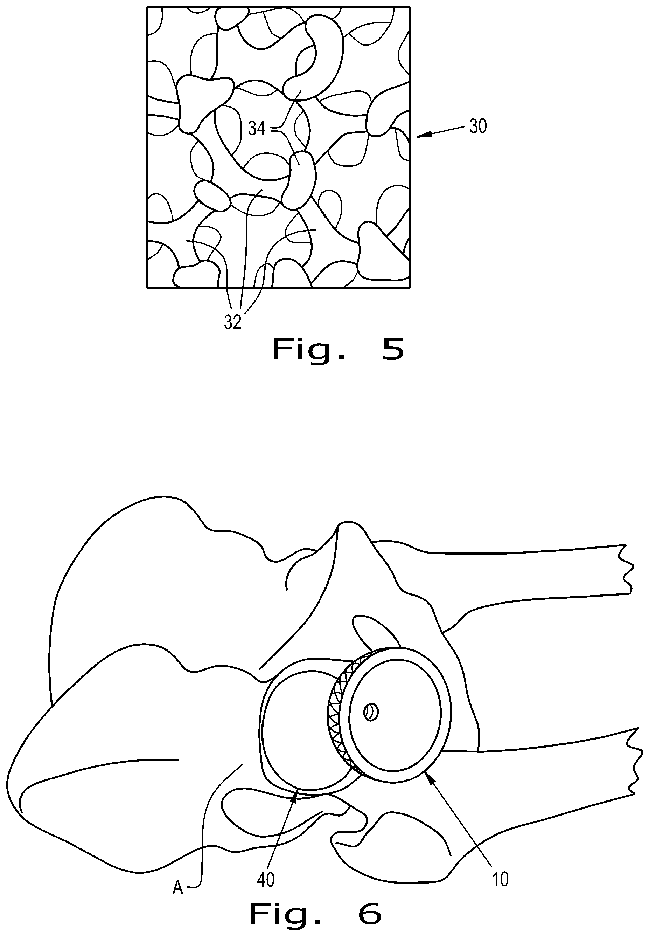

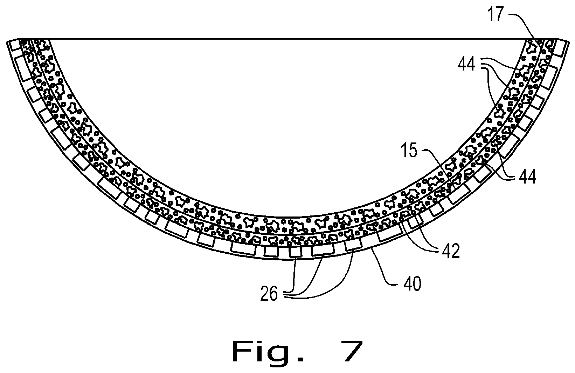

Orthopaedic implants, such as acetabular cup 10, formed according to the present invention can be implanted in a human or non-human subject using techniques similar to untextured orthopaedic implants. When implanting the acetabular cup 10, for example, and referring now to Fig. 6, an acetabulum A is prepared using typical surgical techniques of gaining access to and reaming the acetabulum A, producing a prepared anatomical site 40 in the acetabulum A to accept the acetabular cup 10. After the acetabulum A is reamed, the acetabular cup 10 can be pressed into the prepared site 40 of the acetabulum A to press-fit the acetabular cup 10 into the prepared acetabulum A. As the acetabular cup 10 is being pressed into the prepared acetabulum A, the islands 26 of the porous material 14 scrape against one or more surfaces of the prepared acetabulum A and shear off bone tissue and other biological material, which is directed into the pores 19, 21 of the porous material 14 to pack bone material into the pores 19, 21. While only the pores 19, 21 of two porous material layers 15, 17 are shown as being filled with recently sheared biological material, it should be appreciated that more than two porous material layers of the porous material 14 may be filled with recently sheared biological material as the implant 10 is pressed into the prepared acetabulum A. In addition to the bone material, blood and other biological substances, such as stem cells and growth factors, from the surrounding surgical site can also be pushed into the pores 19, 21 as the acetabular cup 10 is pressed into the prepared acetabulum A, filling the pores 19, 21 of the porous material 14 with a variety of uncultured biological materials, such as bone tissue 42 and stem cells 44, that promote ingrowth of tissue into the pores 19, 21, as can be seen in Fig. 7. The tissue-growth friendly environment created in the pores 19, 21 of the porous material 14 combined with the repair response that is provoked by shearing the surface of the prepared acetabulum A creates a synergy that encourages rapid tissue ingrowth into the pores 19, 21 of the porous material 14 and surprisingly rapid, solid fixation of the orthopaedic implant 10 to the bone. It should therefore be appreciated that texturing the outermost surface of an orthopaedic implant according to the present invention can be applied to a wide variety of orthopaedic implants that will press against one or more bones during implantation in order to form both an ingrowth-friendly environment in the pores of the orthopaedic implant as well as a damaged bone surface that will provoke the natural repair response of the sheared bone(s).

While this invention has been described with respect to at least one embodiment, the present invention can be further modified within the scope of the invention. This application is therefore intended to cover any variations or adaptations of the invention using its general principles. Further, this application is intended to cover such departures from the present disclosure as come within known or customary practice in the art to which this invention pertains as defined by the appended claims.

## Claims

1. An orthopaedic implant (10), comprising:
- an implant body (12) having an outer surface; and
- a textured porous material (14) attached to said outer surface and having a plurality of pores and a plurality of islands extending away from said outer surface,
wherein said textured porous material (14) comprises a plurality of struts (24) defining said plurality of pores (19, 21) there between, said plurality of islands (26) being connected to said plurality of struts (24), **characterized in that** said plurality of islands are configured to shear biological tissue during implantation, wherein the textured porous material comprises a texture configured to shear bone material or other biological tissue at the implantation side when the orthopaedic implant is implanted and direct the sheared tissue into at least one of said plurality of pores (19, 21).

2. The orthopaedic implant according to claim 1, wherein the other biological tissue is blood and/or stem cells.

3. The orthopaedic implant according to at least claim 1 or 2, wherein at least one of said plurality of islands (26) includes a curved edge.

4. The orthopaedic implant according to at least one of the claims 1 to 3,
wherein said textured porous material (14) includes a first porous material layer defining an outer porous layer and a second porous material layer between said outer porous layer and said outer surface of said implant body (12).

5. The orthopaedic implant according to claim 4, wherein said first porous material layer (30) includes a first plurality of pores (35) and said second porous material (33) layer includes a second plurality of pores (37) which do not completely overlap said first plurality of pores.

6. The orthopaedic implant according to claim 5, wherein said first porous material layer (30) defines a first layer thickness and each of said plurality of islands define an island thickness which is greater than said first layer thickness.

7. The orthopaedic implant according to claim 2, wherein said textured porous material (14) comprises a plurality of porous material layers (30, 33) each having a plurality of pores (35, 37) and said bone material or other biological tissue is packed into pores of at least two of said plurality of porous material layers.

8. The orthopaedic implant according to at least one of the claims 1 to 7, wherein said plurality of islands (26) comprises a first island and a second island which has a different shape than said first island and/or each of said plurality of islands has a maximum dimension no greater than 600 microns.

9. The orthopaedic implant according to claim 1, wherein said islands comprise a shearing material with a hardness greater than cortical bone.

10. The orthopaedic implant according to at least one of the claims 1 to 9 wherein said implant body defines a semi-spherical shape.

## Patentansprüche

1. Orthopädisches Implantat (10), umfassend:
- einen Implantatkörper (12) mit einer Außenfläche; und
- ein strukturiertes poröses Material (14), das an der Außenfläche angebracht ist und eine Vielzahl von Poren und eine Vielzahl von Inseln, die sich von der Außenfläche weg erstrecken, aufweist,
wobei das strukturierte poröse Material (14) eine Vielzahl von Streben (24) umfasst, die die Vielzahl von Poren (19, 21) dazwischen definiert, wobei die Vielzahl von Inseln (26) mit der Vielzahl von Streben (24) verbunden ist, **dadurch gekennzeichnet, dass** die Vielzahl von Inseln dazu konfiguriert ist, biologisches Gewebe während der Implantation zu schneiden, wobei das strukturierte poröse Material eine Struktur umfasst, die dazu konfiguriert ist, Knochenmaterial oder anderes biologisches Gewebe an der Implantationsstelle zu schneiden, wenn das orthopädische Implantat implantiert wird, und das geschnittene Gewebe in mindestens eine der Vielzahl von Poren (19, 21) zu leiten.

2. Orthopädisches Implantat nach Anspruch 1, wobei es sich bei dem anderen biologischen Gewebe um Blut und/oder Stammzellen handelt.

3. Orthopädisches Implantat nach mindestens einem der Ansprüche 1 oder 2, wobei mindestens eine der Vielzahl von Inseln (26) eine gekrümmte Kante aufweist.

4. Orthopädisches Implantat nach mindestens einem der Ansprüche 1 bis 3, wobei das strukturierte poröse Material (14) eine erste Schicht aus porösem Material, die eine äußere poröse Schicht definiert, und eine zweite Schicht aus porösem Material zwischen der äußeren porösen Schicht und der Außenfläche des Implantatkörpers (12) beinhaltet.

5. Orthopädisches Implantat nach Anspruch 4, wobei die erste Schicht (30) aus porösem Material eine Vielzahl von Poren (35) beinhaltet und die zweite Schicht (33) aus porösem Material eine zweite Vielzahl von Poren (37) beinhaltet, die die erste Vielzahl von Poren nicht vollständig überlappt.

6. Orthopädisches Implantat nach Anspruch 5, wobei die erste Schicht (30) aus porösem Material eine Dicke der ersten Schicht definiert und jede der Vielzahl von Inseln eine Inseldicke definiert, die größer als die Dicke der ersten Schicht ist.

7. Orthopädisches Implantat nach Anspruch 2, wobei das strukturierte poröse Material (14) eine Vielzahl von Schichten (30, 33) aus porösem Material jeweils mit einer Vielzahl von Poren (35, 37) umfasst und das Knochenmaterial oder das andere biologische Gewebe in Poren von mindestens zwei der Vielzahl von Schichten aus porösem Material eingeschlossen ist.

8. Orthopädisches Implantat nach mindestens einem der Ansprüche 1 bis 7, wobei die Vielzahl von Inseln (26) eine erste Insel und eine zweite Insel, die eine andere Form als die erste Insel aufweist, umfasst, und/oder jede der Vielzahl von Inseln eine maximale Abmessung von nicht mehr als 600 Mikrometern aufweist.

9. Orthopädisches Implantat nach Anspruch 1, wobei die Inseln ein Schneidmaterial mit einer Härte umfassen, die größer als der kortikale Knochen ist.

10. Orthopädisches Implantat nach mindestens einem der Ansprüche 1 bis 9, wobei der Implantatkörper eine Halbkreisform definiert.

## Revendications

1. Implant orthopédique (10), comprenant :
- un corps d'implant (12) ayant une surface externe ; et
- un matériau poreux texturé (14) fixé à ladite surface externe et comportant une pluralité de pores et une pluralité d'îlots s'étendant à l'opposé de ladite surface externe,
dans lequel ledit matériau poreux texturé (14) comprend une pluralité d'entretoises (24) définissant ladite pluralité de pores (19, 21) entre elles, ladite pluralité d'îlots (26) étant reliée à ladite pluralité d'entretoises (24),
**caractérisé en ce que** ladite pluralité d'îlots est configurée pour cisailler un tissu biologique pendant une implantation, dans lequel
le matériau poreux texturé comprend une texture configurée pour cisailler un matériau osseux ou un autre tissu biologique au niveau du côté implantation lorsque l'implant orthopédique est implanté et pour diriger le tissu cisaillé dans au moins l'un de ladite pluralité de pores (19, 21).

2. Implant orthopédique selon la revendication 1, dans lequel l'autre tissu biologique est du sang et/ou des cellules souches.

3. Implant orthopédique selon au moins la revendication 1 ou 2, dans lequel au moins l'un de ladite pluralité d'îlots (26) inclut un bord incurvé.

4. Implant orthopédique selon au moins l'une des revendications 1 à 3,
dans lequel ledit matériau poreux texturé (14) inclut une première couche de matériau poreux définissant une couche poreuse externe et une deuxième couche de matériau poreux entre ladite couche poreuse externe et ladite surface externe dudit corps d'implant (12).

5. Implant orthopédique selon la revendication 4, dans lequel ladite première couche de matériau poreux (30) inclut une première pluralité de pores (35) et ladite deuxième couche de matériau poreux (33) inclut une deuxième pluralité de pores (37) qui ne chevauchent pas complètement ladite première pluralité de pores.

6. Implant orthopédique selon la revendication 5, dans lequel ladite première couche de matériau poreux (30) définit une première épaisseur de couche et chacun de ladite pluralité d'îlots définit une épaisseur d'îlot qui est supérieure à ladite première épaisseur de couche.

7. Implant orthopédique selon la revendication 2, dans lequel ledit matériau poreux texturé (14) comprend une pluralité de couches de matériau poreux (30, 33) ayant chacune une pluralité de pores (35, 37) et ledit matériau osseux ou ledit autre tissu biologique est compacté dans les pores d'au moins deux de ladite pluralité de couches de matériau poreux.

8. Implant orthopédique selon au moins l'une des revendications 1 à 7,
dans lequel ladite pluralité d'îlots (26) comprend un premier îlot et un deuxième îlot qui a une forme différente de celle dudit premier îlot et/ou chacun de ladite pluralité d'îlots a une dimension maximale non supérieure à 600 microns.

9. Implant orthopédique selon la revendication 1, dans lequel lesdits îlots comprennent un matériau de cisaillement ayant une dureté supérieure à celle de l'os cortical.

10. Implant orthopédique selon au moins l'une des revendications 1 à 9,
dans lequel ledit corps d'implant définit une forme hémisphérique.
